# EUROPÄISCHE PATENTSCHRIFT

(11) **EP 1 362 610 B1**
(45) Veröffentlichungstag und Bekanntmachung des Hinweises auf die Patenterteilung: **17.05.2006**
(21) Anmeldenummer: 02010731.4
(22) Anmeldetag: 14.05.2002
(51) Int. Cl.: A61M 16/08

(54) **Vorrichtung zur Ableitung von Atemgas**
Device for evacuating respiratory gas
Dispositif pour l'évacuation de gaz respiratoire

(43) Veröffentlichungstag der Anmeldung: 19.11.2003
(73) Patentinhaber: Weinmann Geräte für Medizin GmbH & Co. KG, 22525 Hamburg (DE)
(72) Erfinder: Eifler, Martin, 25358 Horst (DE)
(74) Vertreter: Klickow, Hans-Henning

(56) Entgegenhaltungen:
- EP-A- 1 138 340
- US-A- 5 937 851
- US-A- 6 112 745

## Beschreibung

Die Erfindung betrifft eine Vorrichtung zur Ableitung von Atemgas, die ein Innenteil und ein Außenteil aufweist und bei der das Innenteil und das Außenteil mindestens bereichsweise gemeinsam mindestens einen Ausströmspalt begrenzen, sowie bei der das Innenteil aus einem Sockelsegment und einem Anschlußsegment ausgebildet ist und das Sockelsegment und das Anschlußsegment relativ zueinander mit einem Abstand angeordnet und von mindestens zwei Distanzelementen miteinander verbunden sind, zwischen denen sich ein Durchlaß zur Verbindung eines Innenraumes des Innenteiles mit dem Ausströmspalt erstreckt, sowie bei der mindestens eines der Distanzelemente als ein Radialsteg ausgebildet ist, der sich bezüglich einer Strömungslängsachse des Innenteiles in einer radialen Richtung erstreckt, sowie bei der das Innenteil im Bereich seiner dem Außenteil zugewandten Ausdehnung mit einem sich erweiternden Endsegment versehen ist.

Eine Vorrichtung zur Ableitung von Atemluft wird beispielsweise in der US 59 37 851 beschrieben. Die Atemluft wird hierbei zunächst einer Zwischenkammer zugeführt und strömt aus dieser Zwischenkammer in den Ausströmspalt. Die Zwischenkammer dient zur radialen Strömungsverteilung, vermindert die Strömungsgeschwindigkeit und verringert eine Geräuschübertragung durch die Einkapselung des räumlichen Bereiches, in dem wesentliche Anteile der Geräusche entstehen. Nachteilig ist jedoch, daß ein relativ hoher Strömungswiderstand erzeugt wird, und daß eine relativ komplizierte Geometrie der einzelnen Bauteile vorliegt, die zu fertigungstechnischen Problemen führt. Darüber hinaus führt die Zwischenkammer zu Verwirbelungen, die selbst wieder Geräusche erzeugen.

Aus der US 6,112,745 ist es bereits bekannt, eine Vorrichtung zur Ableitung von Atemgas mit einem Innenteil und einem Außenteil zu konstruieren, wobei das Innenteil und daß Außenteil gemeinsam einen Ausströmspalt begrenzen. Das Innenteil besteht aus einem Sockelsegment sowie einem Anschlußsegment, die im Bereich einer ringförmigen Einschnürung miteinander verbunden sind. Die Einschnürung bildet im Bereich ihrer dem Außenteil zugewandten Ausdehnung einen ringförmigen Ausgleichsraum aus. Im Bereich der Einschnürung sind ebenfalls Durchlaßausnehmungen angeordnet, die in den Ausgleichsraum einmünden. zwischen den Durchlaßausnehmungen ererstrecken sich zwei Distanzelemente, die einen Teil der ringförmigen Einschnürung ausbilden.

Eine Vorrichtung zur Ableitung von Atemlegt gemäß Artikel 54(3) wird auch in der EP 1 3144 46 beschrieben.

Aufgabe der vorliegenden Erfindung ist es, eine Vorrichtung der einleitend genannten Art derart zu konstruieren, daß eine gleichmäßige Ausströmung mit geringem Strömungswiderstand und hoher Geräuschdämpfung erreicht wird.

Diese Aufgabe wird erfindungsgemäß dadurch gelöst, daß sich der Durchlaß ausgehend vom Innenraum in Richtung auf den Ausströmspalt erweitert und daß das Anschlußsegment mindestens zwei Federlaschen mit Rastungen trägt, die das Außenteil relativ zum Innenteil fixieren.

Durch die Verbindung des Sockelsegmentes und des Anschlußsegmentes durch die Distanzelemente werden Durchlaßbereiche bereitgestellt, die das Austreten des Atemgases mit geringem Strömungswiderstand durch den Ausströmspalt hindurch unterstützen. Insbesondere wird erreicht, daß eine sehr gleichmäßige Strömung vorgegeben wird und daß Strömungsturbulenzen, die bei der Verwendung eines zwischengeschalteten Ausgleichsraumes erzeugt würden, zuverlässig vermieden sind. Insbesondere während einer Ausatmungsphase kann im gesamten Umfangsbereich eine sehr gleichmäßige Strömung ohne nennenswerte Verwirbelungen und somit auch mit geringen Strömungsgeräuschen erreicht werden. Diese günstigen Strömungseigenschaften liegen aber auch in den weiteren Betriebsphasen vor.

Ebenfalls ist es möglich, durch die erläuterte Konstruktion eine gleichmäßige Ausströmung nur in einem vorgegebenen Teil des Umfangsbereiches zu realisieren und ein restliches Umfangssegment zu verschließen. Hierdurch kann es insbesondere vermieden werden, daß eine Strömung in Richtung auf den Körper des Patienten erfolgt. Ein derartiges teilweises Verschließen des Umfangsbereiches hat den Vorteil, daß der Patient die Verwendung der Vorrichtung als angenehmer empfindet, da eine Kühlwirkung und eine Austrocknung durch eine direkte Anströmung des Körpers des Patienten vermieden werden.

Eine gleichmäßige Strömungsführung wird dadurch unterstützt, daß mindestens eines der Distanzelemente als ein Radialsteg ausgebildet ist, der sich bezüglich einer Strömungslängsachse des Innenteiles in einer radialen Richtung erstreckt.

Ein guter Kompromiß zwischen einer ausreichenden mechanischen Festigkeit und einer günstigen Herstellbarkeit wird dadurch erreicht, daß in einer Umfangsrichtung des Innenteiles vier Radialstege im wesentlichen äquidistant relativ zueinander angeordnet sind.

Eine andere variante besteht darin, daß mindestens eines der Distanzelemente als ein Quersteg ausgebildet ist, der sich bezüglich einer Strömungslängsachse des Innenteiles quer zu einer radialen Richtung erstreckt. Insbesondere ist daran gedacht, daß sich der Quersteg mit seiner Außenkontur an das Außenteil anschmiegt, um hierdurch die Bildung einer Kammer oder eines Spaltes möglichst zu vermeiden oder zumindest einen verbleibenden Abstand zu reduzieren. Auch hierdurch kann eine Geräuschentwicklung durch Strömungsturbulenzen vermieden bzw. vermindert werden.

Auch bei einer derartigen Konstruktion besteht ein günstiger Kompromiß zwischen unterschiedlichen Anforderungen darin, daß in einer Umfangsrichtung des Innenteiles vier Querstege im wesentlichen äquidistant relativ zueinander angeordnet sind.

Eine weitere Variante zur Erreichung einer günstigen Strömungsführung sowie zur Gewährleistung einer hohen mechanischen Stabilität besteht darin, daß als Distanzelement sowohl mindestens ein Radialsteg als auch mindestens ein Quersteg verwendet ist.

Beispielsweise ist daran gedacht, daß zwei Radialstege und zwei Querstege verwendet sind.

Ein Auftreten von Verwirbelungen kann weitgehend dadurch vermieden werden, daß mindestens eines der Distanzelemente als keilförmiges Element ausgebildet ist, das sich bezüglich einer Strömungslängsachse des Innenteiles in einer radialen Richtung und mit zunehmendem Abstand zur Strömungslängsachse erweitert.

Eine Ausführungsvariante besteht darin, daß in einer Umfangsrichtung des Innenteiles vier keilförmige Elemente im wesentlichen äquidistant relativ zueinander angeordnet sind.

Eine sehr gleichmäßige Materialverteilung der Vorrichtung bei gleichzeitig sehr gleichmäßiger Strömungsverteilung kann dadurch erreicht werden, daß entlang der Umfangsrichtung des Innenteiles eine Mehrzahl von Distanzelementen angeordnet sind, die jeweils sowohl in Umfangsrichtung als auch in einer radialen Richtung mit einer geringen Dimensionierung versehen sind.

Typischerweise ist insbesondere daran gedacht, daß mindestens sechs Distanzelemente verwendet sind.

Zur Unterstützung einer einfachen Montierbarkeit und Demontierbarkeit ist daran gedacht, daß das Anschlußelement mindestens zwei Federlaschen trägt.

Eine auch größeren Belastungen widerstehende Verbindung zwischen dem Innenteil und dem Außenteil kann dadurch erreicht werden, daß die Federlaschen mit Rastungen versehen sind, die das Außenteil relativ zum Innenteil fixieren.

In der Zeichnung sind Ausführungsbeispiele der Erfindung schematisch dargestellt. Es zeigen:
- Fig. 1:: eine perspektivische vereinfachte Darstellung einer Beatmungseinrichtung, die aus einer Versorgungseinheit, einem Verbindungsschlauch sowie einer Beatmungsmaske ausgebildet ist,
- Fig. 2:: einen vergrößerten Querschnitt durch ein Ausatmungselement, das im Bereich des verbindungsschlauches zwischen der Versorgungseinheit und der Beatmungsmaske anordbar ist,
- Fig. 3:: eine Seitenansicht eines Ausströmelementes mit radialen Stegen,
- Fig. 4: einen Horizontalschnitt gemäß Schnittlinie A-A in Figur 3 zur Veranschaulichung der Strömungswege,
- Fig. 5: eine perspektivische Darstellung des Ausströmelementes gemäß Fig. 3 nach einem Abnehmen des Außenteiles,
- Fig. 6: eine Seitenansicht eines Ausströmelementes mit radialen Stegen und Querstegen,
- Fig. 7: einen Horizontalschnitt gemäß Schnittlinie A-A in Fig. 6 zur Veranschaulichung der Strömungswege,
- Fig. 8: eine perspektivische Darstellung des Ausströmelementes gemäß Fig. 6 nach einem Abnehmen des Außenteiles
- Fig. 9: eine Seitenansicht eines Ausströmelementes mit Querstegen,
- Fig. 10: einen Horizontalschnitt gemäß Schnittlinie A-A in Figur 9 zur Veranschaulichung der Strömungswege,
- Fig. 11: eine perspektivische Darstellung des Ausströmelementes gemäß Fig. 9 nach einem Abnehmen des Außenteiles
- Fig. 12: eine Seitenansicht eines Ausströmelementes mit keilförmigen Stegen,
- Fig. 13: einen Horizontalschnitt gemäß der Schnittlinie A-A in Figur 12 zur Veranschaulichung der Strömungswege,
- Fig. 14: eine perspektivische Darstellung des Ausströmelementes gemäß Fig. 12 nach einem Abnehmen des Außenteiles
- Fig. 15: eine Seitenansicht eines Ausströmelementes mit Stegen, die eine kleine Querschnittfläche aufweisen,
- Fig. 16: einen Horizontalschnitt gemäß Schnittlinie A-A in Figur 15 zur Veranschaulichung der Strömungswege und
- Fig. 17: eine perspektivische Darstellung des Ausströmelementes gemäß Fig. 15 nach einem Abnehmen des Außenteiles

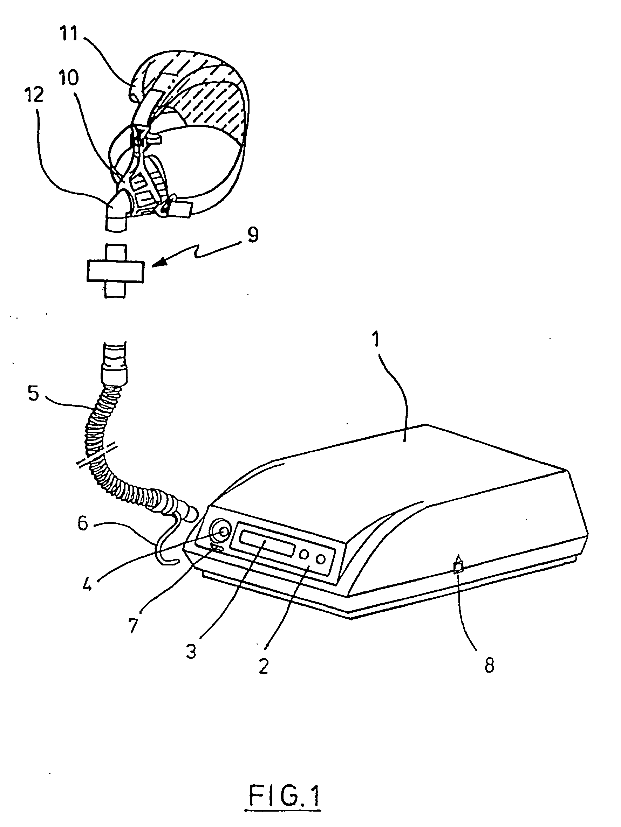

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung des Verbindungsschlauches (5) ist ein Ausatmungselement (9) angeordnet.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) einen Anschlußstutzen (12) auf.

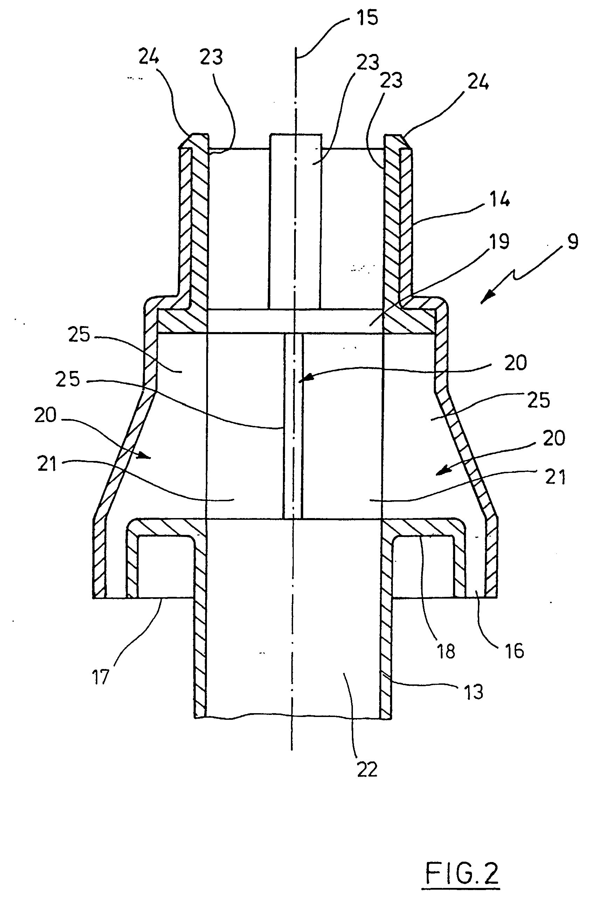

Fig. 2 zeigt einen Querschnitt durch das Ausatmungselement (9) in einer vergrößerten Darstellung. Es ist erkennbar, daß das Ausatmungselement (9) aus einem Innenteil (13) sowie einem Außenteil (14) ausgebildet ist. Das Außenteil (14) erstreckt sich in einem Teilbereich seiner Ausdehnung entlang einer Strömungslängsachse (15) oder in einem Winkel zur Strömungslängsachse (15) mit einem Abstand zum Innenteil (13) und begrenzt hierdurch gemeinsam mit dem Innenteil (13) einen Ausströmspalt (16).

Bei der dargestellten Ausführungsform weisen das Innenteil (13) und das Außenteil (14) eine im wesentlichen konzentrische Gestaltung bezüglich der Strömungslängsachse (15) auf. Im Bereich seiner der Beatmungsmaske (10) zuwendbaren Ausdehnung besitzt das Außenteil (14) einen Außendurchmesser, der im wesentlichen dem Außendurchmesser des Innenteiles (13) im Bereich dessen dem Verbindungsschlauch (5) zuwendbaren Ausdehnung entspricht. In Richtung auf das Innenteil (13) erweitert sich das Außenteil (14). Das Innenteil (13) ist im Bereich seiner dem Außenteil (14) zugewandten Ausdehnung ebenfalls mit einem sich erweiterndem Endsegment (17) ausgestattet.

Fig. 2 veranschaulicht darüber hinaus, daß das Innenteil (13) aus einem Sockelsegment (18) und einem Anschlußsegment (19) ausgebildet ist, die in Richtung der Strömungslängsachse (15) mit einem Abstand relativ zueinander angeordnet sind. Das Sockelsegment (18) und das Anschlußsegment (19) sind durch Distanzelemente (20) miteinander verbunden, die quer zur Strömungslängsachse (15) Durchlässe (21) begrenzen. Die Durchlässe (21) verbinden einen Innenraum (22) des Innenteiles (13) mit dem Ausströmspalt (16).

Das Anschlußsegment (19) trägt Federlaschen (23), die mit Rastungen (24) versehen sind und das Außenteil (14) relativ zum Innenteil (13) fixieren.

Bei der in Fig. 2 dargestellten Ausführungsform sind die Distanzelemente (20) als Radialstege (25) ausgebildet.

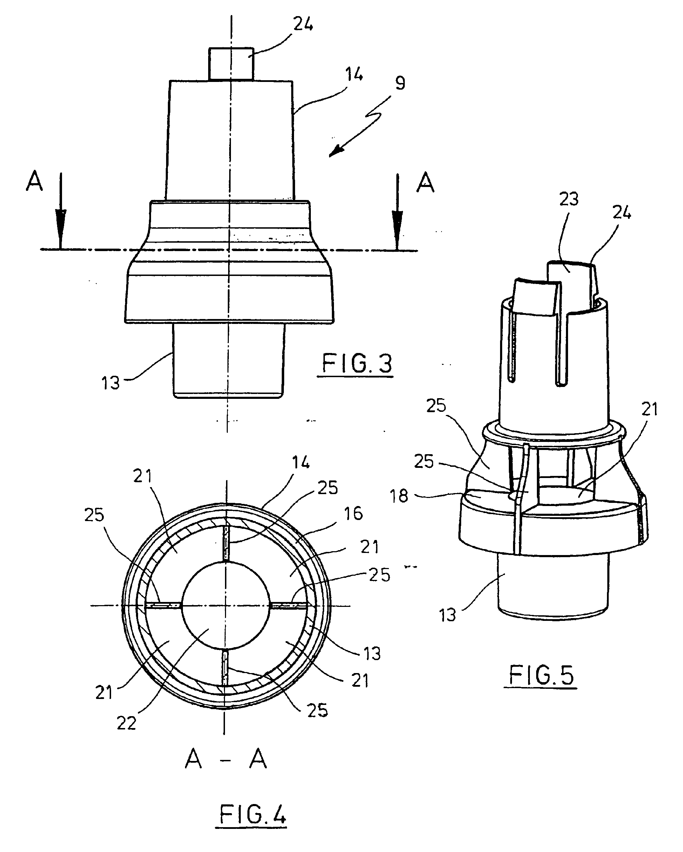

Fig. 3 zeigt die Ausführungsform gemäß Fig. 2 in einer Seitenansicht. Es ist insbesondere die glockenartige Erweiterung der Außenkontur in Richtung der Strömungslängsachse (15) erkennbar.

Aus der schematischen Querschnittdarstellung in Fig. 4 ist erkennbar, daß aufgrund der räumlichen Anordnung der Radialstege (25) eine Erweiterung der Durchlässe (21) ausgehend vom Innenraum (22) in Richtung auf den Ausströmspalt (16) realisiert ist. Hierdurch wird eine sehr gleichmäßige Strömung in Richtung auf den Ausströmspalt (16) unterstützt.

Fig. 5 zeigt die Ausführungsform gemäß Fig. 2 und Fig. 3 in einer perspektivischen Darstellung. Es ist insbesondere erkennbar, daß vier Radialstege (25) verwendet sind, die seitlich vier Durchlässe (21) begrenzen.

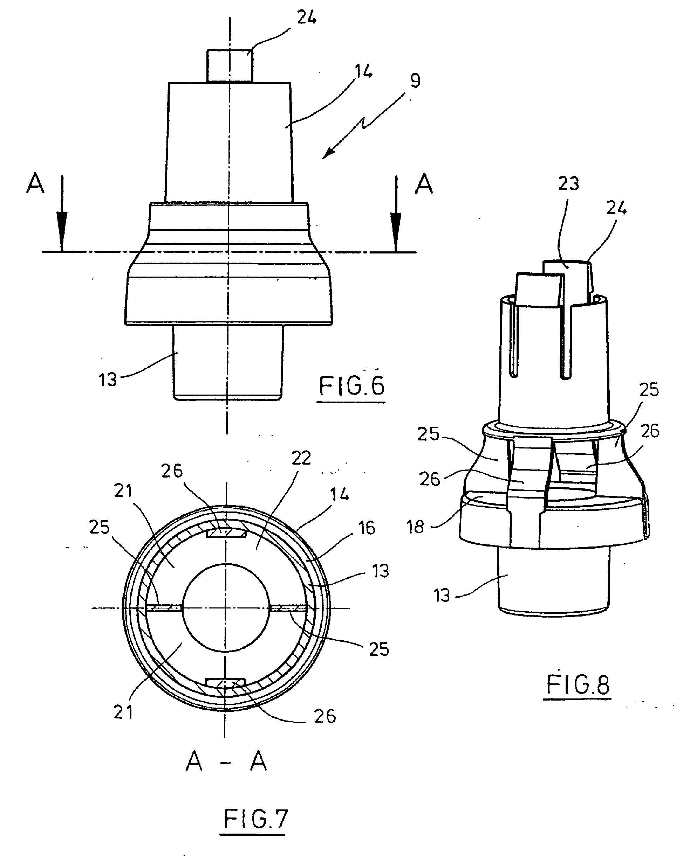

Fig. 6 bis Fig. 8 zeigen eine Ausführungsform, bei der zwei der Distanzelemente (20) als Radialstege (25) und zwei weitere Distanzelemente (20) als Querstege (26) ausgebildet sind. Die Querstege (26) können dabei mit einer recheckförmigen Querschnittfläche versehen sein, insbesondere ist aber auch daran gedacht, eine Außenkontur der Querstege (26) im wesentlichen parallel zur Innenbegrenzung des Außenteiles (14) verlaufen zu lassen. Ein Abstand zwischen den Distanzelementen (20) und dem Außenteil (14) kann beispielsweise 0,3 Millimeter betragen.

Durch die Kombination der Radialstege (25) und der Querstege (26) ergeben sich im wesentlichen zwei große Verbindungsräume zur Überleitung des Atemgases vom Innenraum (22) zum Ausströmspalt (16).

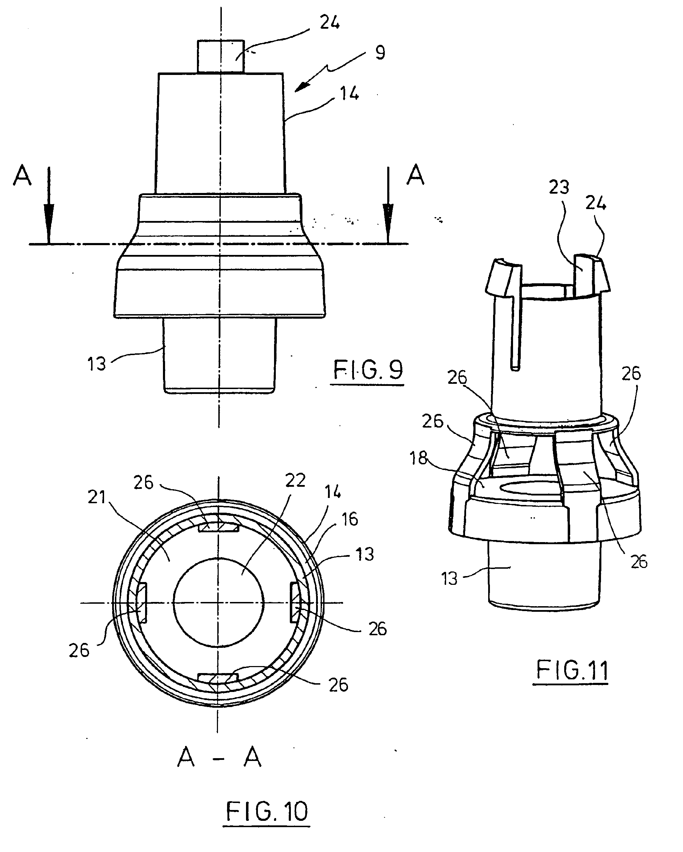

Bei der Ausführungsform gemäß Fig. 9 bis Fig. 11 werden als Distanzelemente (20) ausschließlich Querstege (26) verwendet. Es ergibt sich hierdurch ein gemeinsamer großer Überleitungsraum zur Verbindung des Innenraumes (22) mit dem Ausströmspalt (16). Auch bei dieser Ausführungsform ist es möglich, zwischen den Distanzelementen (20) und dem Außenteil (14) beispielsweise einen Abstand von etwa 0,3 Millimetern zu realisieren.

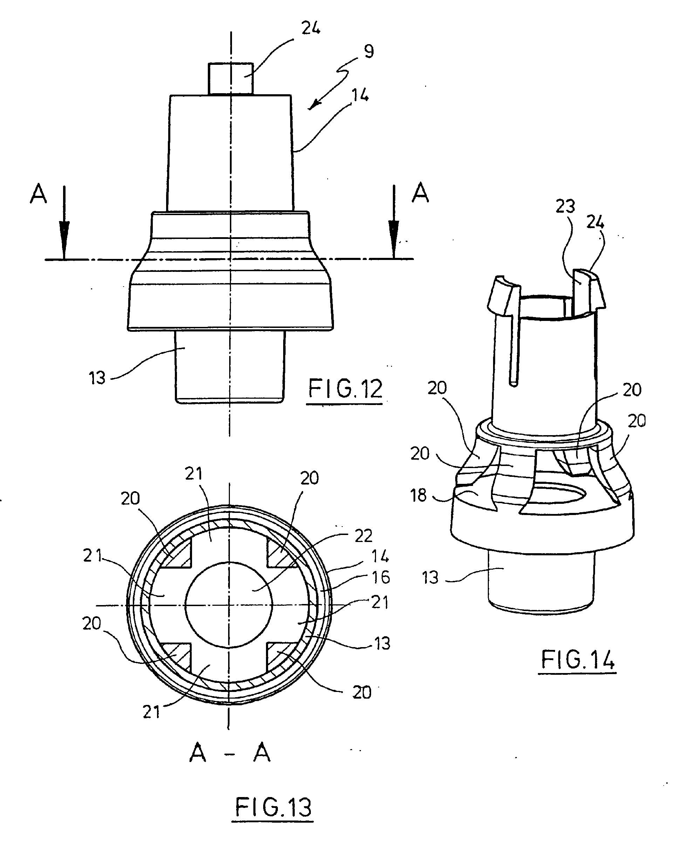

Fig. 12 und Fig. 14 zeigen eine Ausführungsform, bei der die Distanzelemente (20) eine Gestaltung derart aufweisen, daß die zwischen den Distanzelementen (20) angeordneten Durchlässe (21) mit im wesentlichen konstanter Breite versehen sind. Dies kann beispielsweise dadurch erreicht werden, daß sich die Distanzelemente (20) in Umfangsrichtung ausgehend vom Innenraum (22) in Richtung auf den Ausströmspalt (16) erweitern. Die Erweiterung kann beispielsweise durch eine in Fig. 13 dargestellte massive Ausführungsform mit keilförmigen Distanzelementen (20) realisiert sein, es ist aber ebenfalls möglich, die Distanzelemente (20) durch entsprechende dünnwandige Begrenzungen zu realisieren, die einen Innenraum umgeben oder zumindest teilweise umschließen. Ein Abstand zwischen den Distanzelementen (20) und dem Außenteil (14) kann wiederum beispielsweise in einer Größenordnung von etwa 0,3 Millimetern realisiert sein.

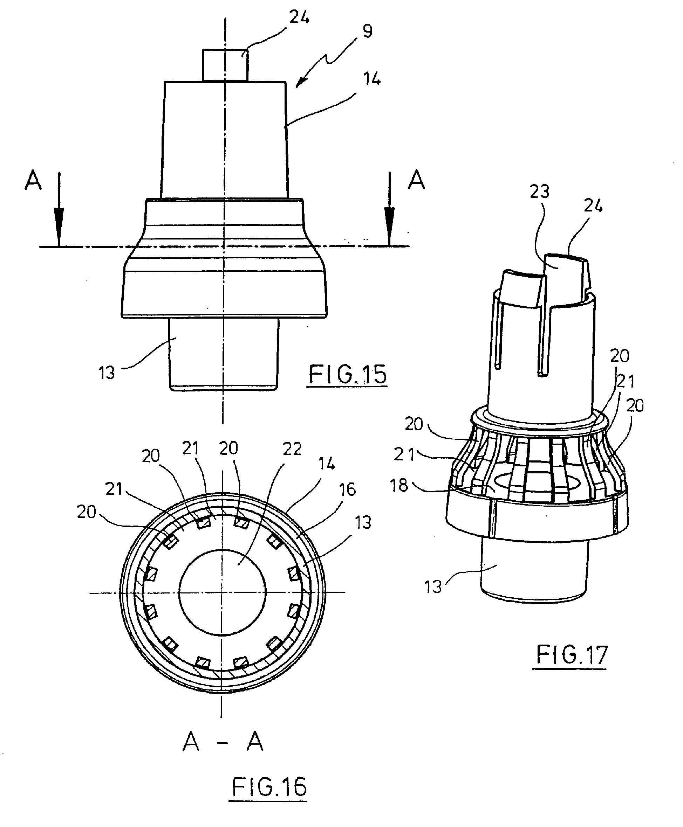

Die Ausführungsform gemäß Fig. 15 bis Fig. 17 zeigt die Anordnung einer Vielzahl von in Umfangsrichtung relativ schmal ausgebildeten Distanzelementen (20). Aufgrund der Vielzahl der Distanzelemente (20) werden ebenfalls eine Vielzahl von Durchlässen (21) realisiert. Bei der dargestellten Ausführungsform werden zwölf Distanzelemente (20) verwendet. Typischerweise beträgt die Anzahl der Distanzelemente (20) bei einer derartigen Realisierung mindestens sechs. Auch bei dieser Ausführungsform kann ein Abstand zwischen den Distanzelementen (20) und dem Außenteil (14) beispielsweise wieder etwa 0,3 Millimeter betragen. Aufgrund der Verwendung der Vielzahl von Distanzelementen (20) und der relativ geringen Dimensionierung der Distanzelemente (20) sowohl in Umfangsrichtung als auch in Richtung auf die Strömungslängsachse (15) wird ein relativ großer Überleitungsraum bereitgestellt, der durch eine Vielzahl von relativ kleinen Durchlässen (21) in den Ausströmspalt (16) übergeleitet ist.

Bei sämtlichen dargestellten Ausführungsformen ist der Ausströmspalt (16) als ein Ringspalt ausgeführt. Grundsätzlich ist es aber auch denkbar, den Ausströmspalt (16) mit einer anderen Gestaltung zu versehen oder in Öffnungssegmente zu unterteilen.

Eine fertigungstechnische Herstellung des Innenteiles (13) und des Außenteiles (14) kann aus einem oder mehreren Kunststoffen erfolgen. Insbesondere ist daran gedacht, die Teile (13, 14) spritzgußtechnisch herzustellen.

Bei den in den Figuren dargestellten Ausführungsbeispielen besteht das Anschlußsegment (19) aus einem ringartigen Bauelement, das zur Stabilisierung alle Distanzelemente (20) miteinander verbindet und das die Federlaschen (23) trägt. Gemäß einer abgewandelten Ausführungsform ist es aber auch möglich, das Anschlußsegment (19) aus voneinander getrennten Segmenten auszubilden und hierdurch auf eine steife Verbindung der Distanzelemente (20) im Bereich ihrer dem Sockelsegment (18) abgewandten Ausdehnung zu verzichten.

Bei einer Ausführungsform mit segmentiertem Anschlußsegment (19) ist insbesondere daran gedacht, die einzelnen Bereiche des Anschlußsegmentes (19) als Verlängerungen der Distanzelemente (20) zu realisieren. Zumindest einige dieser Teile der Anschlußsegmente (19) bilden hierbei dann lediglich Übergangsbereiche zwischen den Distanzelementen (20) und den Federlaschen (23) aus. Es ist aber nicht erforderlich, jedem der Distanzelemente (20) eine separate Federlasche zuzuordnen. Beispielsweise können bei der Verwendung von vier Distanzelementen (20) zwei Distanzelemente (20) in Federlaschen (23) übergeleitet werden, die vorzugsweise einander gegenüberliegend angeordnet sind.

## Patentansprüche

1. Vorrichtung zur Ableitung von Atemgas, die ein Innenteil (13) und ein Außenteil (14) aufweist und bei der das Innenteil (13) und das Außenteil (14) mindestens bereichsweise gemeinsam mindestens einen Ausströmspalt (16) begrenzen, sowie bei der das Innenteil (13) aus einem Sockelsegment (18) und einem Anschlußsegment (19) ausgebildet ist und das Sokkelsegment (18) und das Anschlußsegment (19) relativ zueinander mit einem Abstand angeordnet und von mindestens zwei Distanzelementen (20) miteinander verbunden sind, zwischen denen sich ein Durchlaß (21) zur Verbindung eines Innenraumes (22) des Innenteiles (13) mit dem Ausströmspalt (16) erstreckt, sowie bei der mindestens eines der Distanzelemente (20) als ein Radialsteg (25) ausgebildet ist, der sich bezüglich einer Strömungslängsachse (15) des Innenteiles (13) in einer radialen Richtung erstreckt, sowie bei der das Innenteil (13) im Bereich seiner dem Außenteil (14) zugewandten Ausdehnung mit einem sich in Richtung auf das Außenteil (14) erweiternden Endsegment (17) versehen ist, sowie bei der sich der Durchlaß (21) ausgehend vom Innenraum (22) in Richtung auf den Ausströmspalt (21) erweitert, **dadurch gekennzeichnet daß** das Anschlußsegment (19) mindestens zwei Federlaschen (23) mit Rastungen (24) trägt, die das Außenteil (14) relativ zum Innenteil (13) fixieren.

2. Vorrichtung nach Anspruch 1, **dadurch gekennzeichnet, daß** in einer Umfangsrichtung des Innenteiles (13) vier als Radialstege (25) ausgebildete Distanzelemente (20) im wesentlichen äquidistant relativ zueinander angeordnet sind.

3. Vorrichtung nach Anspruch 1, **dadurch gekennzeichnet, daß** mindestens eines der Distanzelemente (20) als ein Quersteg (26) ausgebildet ist, der sich bezüglich einer Strömungslängsachse (15) des Innenteiles (13) mit seiner Längsachse quer zu einer radialen Richtung erstreckt.

4. Vorrichtung nach einem der Ansprüche 1 bis 3, **dadurch gekennzeichnet, daß** in einer Umfangsrichtung des Innenteiles (13) vier als Querstege (26) ausgebildete Distanzelemente (20) im wesentlichen äquidistant relativ zueinander angeordnet sind.

5. Vorrichtung nach einem der Ansprüche 1 bis 4, **dadurch gekennzeichnet, daß** als Distanzelement (20) sowohl mindestens ein Radialsteg (25) als auch mindestens ein Quersteg (26) verwendet ist.

6. Vorrichtung nach Anspruch 5, **dadurch gekennzeichnet, daß** zwei als Radialstege (25) ausgebildete Distanzelemente (20) und zwei als Querstege (26) ausgebildete Distanzelemente (20) verwendet sind.

7. Vorrichtung nach Anspruch 1, **dadurch gekennzeichnet, daß** mindestens eines der Distanzelemente (20) als keilförmiges Element ausgebildet ist, das sich bezüglich einer Strömungslängsachse (15) des Innenteiles (13) in einer radialen Richtung und mit zunehmendem Abstand zur Strömungslängsachse erweitert.

8. Vorrichtung nach einem der Ansprüche 1 bis 7, **dadurch gekennzeichnet, daß** in einer Umfangsrichtung des Innenteiles (13) vier als keilförmige Elemente ausgebildete Distanzelemente (20) im wesentlichen äquidistant relativ zueinander angeordnet sind.

9. Vorrichtung nach Anspruch 1, **dadurch gekennzeichnet, daß** entlang der Umfangsrichtung des Innenteiles (13) eine Mehrzahl von Distanzelementen (20) angeordnet sind, die jeweils sowohl in Umfangsrichtung als auch in einer radialen Richtung mit einer geringen Dimensionierung versehen sind.

10. Vorrichtung nach Anspruch 9 **dadurch gekennzeichnet, daß** mindestens sechs Distanzelemente (20) verwendet sind.

## Claims

1. Device for discharging breathing gas, comprising an inner part (13) and an outer part (14) and wherein the inner part (13) and the outer part (14) commonly bound at least certain regions of at least one outlet gap (16), and wherein the inner part (13) is made up of a socket segment (18) and a connector segment (19) and the socket segment (18) and the connector segment (19) are disposed at a distance relative to one another and are connected by means of at least two spacer elements (20) between which a passage (21) for connecting an inner chamber (22) of the inner part (13) to the outlet gap (16) extends, at least one of which spacer elements (20) is provided in the form of a radial web (25) extending in a radial direction with respect to a flow longitudinal axis (15) of the inner part (13), and wherein in the region of its extension directed towards the outer part (14), the inner part (13) is provided with a terminal segment (17) which becomes wider in the direction towards the outer part (14) and the passage (21) becomes wider in the direction towards the outlet gap (21) starting from the inner chamber (22), **characterised in that** the connector segment (19) bears at least two resilient tabs (23) with catches (24) which secure the outer part (14) relative to the inner part (13).

2. Device as claimed in claim 1, **characterised in that** four spacer elements (20) in the form of radial webs (25) are disposed essentially equidistantly relative to one another in a peripheral direction of the inner part (13).

3. Device as claimed in claim 1, **characterised in that** at least one of the spacer elements (20) is provided in the form of a transverse web (26), which extends with its longitudinal axis transversely to a radial direction by reference to a flow longitudinal axis (15) of the inner part (13).

4. Device as claimed in one of claims 1 to 3, **characterised in that** four spacer elements (20) in the form of transverse webs (26) are disposed essentially equidistantly relative to one another in a peripheral direction of the inner part (13).

5. Device as claimed in one of claims 1 to 4, **characterised in that** both at least one radial web (25) and at least one transverse web (26) are used as a spacer element (20).

6. Device as claimed in claim 5, **characterised in that** two spacer elements (20) are provided in the form of radial webs (25) and two spacer elements (20) are provided in the form of transverse webs (26).

7. Device as claimed in claim 1, **characterised in that** at least one of the spacer elements (20) is provided in the form of a wedge-shaped element which becomes wider in a radial direction by reference to a flow longitudinal axis (15) of the inner part (13) and at an increasing distance from the flow longitudinal axis.

8. Device as claimed in one of claims 1 to 7, **characterised in that** four spacer elements (20) in the form of wedge-shaped elements are disposed essentially equidistantly relative to one another in a peripheral direction of the inner part (13).

9. Device as claimed in claim 1, **characterised in that** a plurality of spacer elements (20) is disposed along the peripheral direction of the inner part (13), each of which is of a slim dimension both in the peripheral direction and in a radial direction.

10. Device as claimed in claim 9, **characterised in that** at least six spacer elements (20) are used.

## Revendications

1. Dispositif pour l'évacuation de gaz respiré, qui présente une pièce interne (13) et une pièce externe (14) et pour lequel la pièce interne (13) et la pièce externe (14) limitent au moins partiellement, en commun, une fente d'écoulement (16), ainsi que pour lequel la pièce interne (13) est formée d'un segment de socle (18) et d'un segment de raccord (19) et le segment de socle (18) et le segment de raccord (19) sont disposés à une certaine distance l'un de l'autre et sont reliés l'un à l'autre par au moins deux éléments de distance (20), entre lesquels s'étend un passage (21) pour la connexion d'un espace interne (22) de la pièce interne (13) avec la fente d'écoulement (16), ainsi que pour lequel au moins un des éléments de distance (20) est formé comme une âme radiale, qui s'étend en direction radiale par rapport à un axe longitudinal d'écoulement (15) de la pièce interne (13), ainsi que dans lequel la pièce interne (13) est munie d'un segment terminal (17) s'élargissant en direction de la pièce externe (14) au niveau de son extension dirigée vers la pièce externe (14), **caractérisé en ce que** le passage (21) s'élargit en direction de la fente d'écoulement (16) partant de l'espace interne (22) et **en ce que** le segment de raccord (19) porte au moins deux jumelles de ressort (23) avec arrêts (24), qui fixe la pièce externe (14) par rapport à la pièce interne (13).

2. Dispositif selon la revendication 1, **caractérisé en ce qu'**en direction de la circonférence de la pièce interne (13), sont disposés quatre éléments de distance (20), formés comme des âmes radiales (25), de manière relativement équidistante l'un par rapport à l'autre.

3. Dispositif selon la revendication 1, **caractérisé en ce qu'**au moins un des éléments de distance (20) est formé comme une âme transversale (26), qui s'étend avec sont axe longitudinal transversalement à une direction radiale par rapport à l'axe longitudinal d'écoulement (15) de la pièce interne (13).

4. Dispositif selon l'une des revendications 1 à 3, **caractérisé en ce que** dans une direction de la circonférence de la pièce interne (13), sont disposés quatre éléments de distance (20), formés comme des âmes transversales (26), de manière relativement équidistante l'un par rapport à l'autre.

5. Dispositif selon l'une des revendications 1 à 4, **caractérisé en ce que** comme élément de distance (20), non seulement au moins une âme radiale (25), mais également au moins une âme transversale (26) sont utilisées.

6. Dispositif selon la revendication 5, **caractérisé en ce que** deux éléments de distance (20) formés comme des âmes radiales (25) et deux éléments de distance (20) formés comme des âmes transversales (26) sont utilisés.

7. Dispositif selon la revendication 1, **caractérisé en ce qu'**au moins un des éléments de distance (20) est formé comme un élément cunéiforme, qui s'élargit en direction radiale par rapport à un axe longitudinal d'écoulement (15) de la pièce interne (13) et à une distance croissante de l'axe longitudinal d'écoulement.

8. Dispositif selon l'une des revendications 1 à 7, **caractérisé en ce qu'**en direction de la circonférence de la pièce interne (13), sont disposés quatre éléments de distance (20) formés comme des éléments cunéiformes, de manière essentiellement équidistante l'un par rapport à l'autre.

9. Dispositif selon la revendication 1, **caractérisé en ce que** le long de la direction de la circonférence de la pièce interne (13), sont disposés un certain nombre d'éléments de distance (20), qui sont munis chaque fois, non seulement en direction de la circonférence, mais également en direction radiale, d'une dimension faible.

10. Dispositif selon la revendication 9, **caractérisé en ce que** l'on utilise au moins six éléments de distance (20).
